# EUROPÄISCHE PATENTSCHRIFT

(11) **EP 2 242 463 B1**
(45) Veröffentlichungstag und Bekanntmachung des Hinweises auf die Patenterteilung: **31.08.2016**
(21) Anmeldenummer: 08869027.6
(22) Anmeldetag: 19.12.2008
(51) Int. Cl.: A61F 5/445, A61F 5/448

(54) **VORRICHTUNG FÜR EINEN TEMPORÄREN ILEO- ODER UROSTOMAVERSCHLUSS**
DEVICE FOR A TEMPORARY ILEOSTOMY OR UROSTOMY CLOSURE
DISPOSITIF POUR OBTURATION TEMPORAIRE ILÉO- OU UROSTOMACALE

(30) Priorität: 21.12.2007 DE 102007062133
(43) Veröffentlichungstag der Anmeldung: 27.10.2010
(73) Patentinhaber: Ketek GmbH, 81737 München (DE)
(72) Erfinder: KRATKY, Peter, 82319 Starnberg (DE)
(74) Vertreter: Samson & Partner Patentanwälte mbB
(86) Internationale Anmeldenummer: PCT/EP2008/010907
(87) Internationale Veröffentlichungsnummer: WO 2009/083183

(56) Entgegenhaltungen:
- EP-A- 1 346 711
- WO-A-87/01274
- WO-A-90/07311
- US-A- 6 050 982
- US-A1- 2006 206 069

## Beschreibung

### GEBIET DER ERFINDUNG

Die vorliegende Erfindung betrifft eine Vorrichtung für einen temporären Ileo- oder Urostomaverschluss.

### HINTERGRUND DER ERFINDUNG

Eine Vorrichtung der eingangs genannten Art findet Verwendung bei Personen mit einer chirurgisch hergestellten Öffnung eines Hohlorgans zur Körperoberfläche, z.B. einem künstlichen Darmausgang, einem sog. Stoma. Das Wort Stoma leitet sich aus dem Griechischen ab und wird übersetzt mit "Mund" oder "Öffnung". Durch entsprechende Vorsilben wird in verschiedene Darmausgänge unterschieden. Man spricht bei der Ausleitung des Dünndarms von einem Ileostoma, des Dickdarms von einem Kolostoma und bei der Ausleitung von Urin von einem Urostoma. Beim Kolostoma wird nochmals unterschieden in Descendostoma bzw. Sigmoidostoma und Transversostoma, beim Urostoma zwischen einer Uretherhautfistel und einem Conduit.

Ein Stoma kann auf Zeit (temporär) oder auf Dauer (permanent) angelegt werden. Ein Stoma kann einlumig (endständig bzw. terminal) oder doppellumig (doppelläufig) sein.

Der häufigste Grund für die Anlage eines Stomas ist eine Krebserkrankung des Mastdarms (Rektumcarcinom) oder des Anus (Analcarcinom). Bei der operativen Entfernung des Tumors kann es notwendig sein, dass zudem der Darm-Schließmuskel entfernt und ein künstlicher Darmausgang am Bauch konstruiert werden muss.

Bei einigen Operationsverfahren kann der natürliche Schließmuskel unter Umständen erhalten werden. In diesem Fall wird nur ein vorübergehender künstlicher Darmausgang angelegt, um die Heilung der Nahtstelle am Dickdarm zu gewährleisten (Anastomosenschutz)

Die zweithäufigste Ursache ist die Erkrankung an einer chronisch entzündlichen Darmerkrankung wie dem Morbus Crohn oder der Colitis ulcerosa. Bei diesen Erkrankungen kann man durch eine Stomaanlage Darmabschnitte, die z.B. entzündet sind, für eine gewisse Zeit oder auf Dauer still legen. Muss man den erkrankten Darm teils oder ganz entfernen, kann ebenfalls eine Stomaanlage notwendig werden.

Bei einem Anus praeter des Ileums wird flüssiger, aggressiver Darminhalt durch das Stoma entleert. Die Flüssigkeitsmenge liegt je nach Trink- und Ernährungsgewohnheit bei bis zu ca. 2500ml/d. Man unterscheidet eine endständige und doppelläufige Ileostomie. Welche Art angelegt wird, ist abhängig von der Erkrankung.

Bei einem Anus praeter des Dickdarms entleert sich mehr oder weniger geformter Stuhl durch das Colostoma. Die Gasbildung ist bei Colostomien groß.

Wie bei der Ileostomie unterscheidet man eine endständige und eine doppelläufige Colostomie: Eine endständige Stoma-Anlage wird immer dann angelegt, wenn im Mastdarm oder am After eine Erkrankung besteht, die es erforderlich macht, dass Mastdarm, Anus mit Schließmuskel entfernt werden müssen, bzw. eine Teilentfernung des erkrankten Darmabschnittes erforderlich ist und die neue Darmanastomose erst nach einem zeitlichen Intervall von mehreren Wochen bzw. Monaten durchgeführt werden kann. Ein endgültig verbleibendes Ileostoma ist z.B. nach Proktocolektomie notwendig, wenn eine Rekonstruktion mittels Anastomose mit dem Enddarm aus technischen oder funktionellen Gründen nicht mehr möglich ist. Andere Krankheitsbilder erfordern eine vorübergehende Stilllegung des betroffenen Darmabschnitts, sodass nach dessen Ausheilung, das Stoma wieder zurückverlegt werden kann.

Bei einem Urostoma wird das Stoma zur Ausleitung von Urin geschaffen. Aufgrund verschiedener Erkrankungen des harnableitenden Systems kommt es zum Verlust seiner Funktion. Um diese wieder gewährleisten zu können, sind bewährte Operationsmethoden notwendig, die in der Regel zur künstlichen Harnableitung führen. Die häufigsten Formen der Harnableitung sind das Ileum - Conduit , das Colon - Conduit und in seltenen Fällen noch die Ureterfisteln (Harnleiterhautfisteln).

Es gibt trockene und nasse Urostomata. Beim nassen Stoma wie bei der Ureter-Haut-Fistel wird der von der Blase abgesetzte Harnleiter direkt durch die Bauchdecke gezogen und an der Haut befestigt. Der Harnleiter muss in den meisten Fällen mittels eines dünnen Katheters auf Dauer geschient bleiben, denn das Stoma kann sich leicht verengen und somit den Abfluss behindern.

Bei einem Ileum-Conduit werden die abgesetzten Harnleiter in einen ausgeschalteten Dünndarmanteil eingepflanzt. Dieses Segment wird nippelförmig in die Bauchdecke eingenäht. Bei einem Colon-Conduit werden die abgesetzten Harnleiter in einen ausgeschalteten Dickdarmanteil eingepflanzt. Dieser Anteil wird auch nippelförmig in die Bauchdecke eingenäht.

An alternativen Operationsmethoden sind zur kontinenten Ileostomie, der ileoanale Pouch (J-, S- oder W-Pouch) und die Kocksche Tasche (Kock-Pouch) zu nennen. Nicht in jedem Fall ist eine dieser Operationsmöglichkeiten möglich. Beim ileoanalen Pouch wird als Alternative zu einem Ileostoma, nach Entfernung des Dickdarms, aus den letzten Dünndarmschlingen ein künstliches Reservoir ähnlich einer Tasche erzeugt. Dieses Reservoir hat am unteren Ende eine Öffnung, die mit dem Schließmuskelapparat verbunden wird. Bei erhaltenem Schließmuskel sammelt sich der Stuhl in dem Pouch und kann dann wie zuvor über den After ausgeschieden werden. Bei der schwer verlaufenden Colitis Ulcerosa wird er meist als J-Pouch von sehr erfahrenen Chirurgen angelegt.

Bei der Kockschen Tasche wird ebenfalls aus den unteren Dünndarmschlingen ein künstliches Reservoir ähnlich einer Tasche gebildet. Ein weiteres Stück Darm wird aus dem Dünndarm entnommen und als Verbindung von der Kockschen Tasche zu einer kleinen Öffnung in der Bauchdecke benutzt. Durch die Operationstechnik wird sichergestellt, das während der Füllphase des Reservoirs kein Stuhl durch die Öffnung in der Bauchdecke nach außen gelangen kann, erst durch das Einführen eines Katheters entleert sich das Reservoir. Heute wird der ileoanale Pouch in der Methode der Wahl dem Kock Pouch vorgezogen.

Man schätzt, dass in der westlichen Welt von 1000 Personen eine mit einem Stoma lebt.

Üblicherweise müssen Dünndarmastomata (Ileostoma) und Urostomata in einen Stomabeutel abgeleitet werden. Der dünnflüssige Darminhalt bei Dünndarmableitungen liegt je nach Nahrungszufuhr bei bis zu 2500ml/ Tag. Der Dickdarm ist ausgeschaltet und erfüllt seine normale Funktion (Regulation von Wasser- und Salzhaushalt) nicht mehr. Für den betroffenen Patienten bedeutet dass, das er ständig einen Stomabeutel am Körper tragen muss, in den sich stündlich beim Ileostoma im Schnitt ca. 80-100ml Flüssigkeit entleert. Die Flüssigkeitsmenge kann man durch ein angepasstes Trink- und Essverhalten regulieren, sie darf jedoch im Tagesmittel gewisse Mengen nicht unterschreiten, um Störungen des Wasserhaushaltes und damit u. a. auch eine renale Dysfunktion zu vermeiden (Verlust von Flüssigkeit und Elektrolyten).

Neben cancerogen Erkrankungen des Colons können auch entzündliche Erkrankungen von Dick- und Dünndarm (z.B. Sigmadivertikulitis, Morbus Crohn oder Colitis ulcerosa) zur Notwendigwendigkeit der Anlage eines Dünndarmstomas führen. Gerade Patienten mit Erkrankungen aus den Bereichen Morbus Crohn oder Colitis ulcerosa müssen nicht selten ein temporäres Ileostoma bzw. auch häufig ein entgültiges Ileostoma erhalten. Dies ist notwendig um persistierende Fistelbildungen im Enddarmbereich zur Abheilung zu bringen bzw. bei schlechter Schließmuskelfunktion. Patienten mit entzündlichen Darmerkrankungen sind überwiegend junge Patienten und müssen im betroffenen Fall viele Jahre mit einem Ileostoma leben.

Die Indikation für die Anlage eines Urostomas (Ileum-Conduit bzw. Bricker Blase) ist u. a. bei Vorliegen von Blasen- Harnleitertumoren, Atresien und Missbildungen, Schrumpfblasen (Strahlenschäden), Gynäkologischen Tumoren, neurogenen Blasenentleerungsstörungen, Tumoren des kleinen Beckens und Verletzungen gegeben.

Irrigation ist ein Begriff aus dem Griechischen und bedeutet "Bewässerung" oder "Spülung". Eine solche Spülung des Darms in regelmäßigen Abständen bewirkt dessen regelmäßige Entleerung. Nach der Irrigation sind für 24 bis 48 Stunden keine Ausscheidung zu erwarten, so dass in dieser Zeit keine Beutel, sondern nur eine unauffällige Stomakappe getragen werden kann. Allerdings ist eine Irrigation nur für Stomaträger mit fest geformten Ausscheidungen geeignet und auch dann nur unter bestimmten Voraussetzungen. Für die Irrigation wird ein spezielles Irrigationsset benötigt.

### STAND DER TECHNIK

Zum Schutz der verschiedenen Stomata bei bestimmten Tätigkeiten hat die Industrie eine ganze Reihe von verschiedenen modischen Stomabandagen bzw. Stomamiedern für Damen und Herren entwickelt. In diese Bandagen und Mieder ist meist eine Tasche für den Beutel integriert und soll dem Patienten vermehrt Sicherheit in der Freizeit, Sport und Beruf bieten.

Das Angebot an Produkten für die Stomaversorgung ist nahezu unüberschaubar groß. Überwiegend stellen die verwendeten Produkte eine einteilige Versorgung bereit d.h. die Hautschutzplatte ist fest mit dem Beutel verbunden. Um eine Überbeanspruchung der Haut zu vermeiden, sollten Einteiler höchstens dreimal am Tag gewechselt werden. Einteiler sind sehr flach, tragen wenig auf, sind flexibel und einfach in der Anwendung.

Die zweiteilige Versorgung umfasst eine Basisplatte als Hautschutz und einen zugehörigen Beutel. Auf der Basisplatte ist ein Rastring angebracht, an den der Beutel einfach angerastet werden kann. Die Basisplatte kann mehrere Tage auf der Haut verbleiben und der Beutel dennoch beliebig oft gewechselt werden.

An Beutelarten gibt es geschlossene Beutel, die in der Regel für die Colostomie verwendet werden. Geschlossene Beutel haben in der Regel einen Aktivkohlefilter, der die bei der Verdauung entstehenden Gase geruchsneutral entweichen lässt. Je nach Bedarf kann der geschlossene Beutel etwa zwei bis drei mal täglich gewechselt werden.

Ausstreifbeutel (offene Beutel) werden überwiegend bei der Ileostomie verwendet, da es hier zu überwiegend dünnflüssigen bis breiigen Ausscheidungen kommt. Ileostomiebeutel haben einen Bodenauslaß, der mit einer Klammer verschlossen wird, sodass der Betroffene den Beutel über den Bodenauslass entleeren kann und nicht ständig die gesamte Versorgung wechseln muss. Meist sind diese Beutel ohne integrierten Filter; allerdings werden von einigen Herstellern auch Ausstreifbeutel mit integriertem Filter angeboten.

Stomaträger mit Dünndarm-/Urostomata sind auf Grund der hohen produktiven Flüssigkeitsmenge gezwungen eine ständige Beutelversorgung zu tragen.

Vorrichtungen und Geräte für einen temporären Colostomaverschluss sind an sich bekannt.

Beipielsweise beschreibt U.S. 6,050,982 einen temporären Colostomaverschluss, bei welchem eine Hülse permanent in das Colostoma eingeführt ist. In dieser Hülse befindet sich ein gefalteter Schlauch, der nach Lösung einer Abdeckkappe herausgezogen und als Leitung zum Abfließen des Darminhaltes verwendet werden kann.

Die WO 90/07311, die den nächstliegenden Stand der Technik darstellt, offenbart ebenfalls einen temporären Colostomaverschluss mit abnehmbarem Deckel und darin integriertem Beutel. Auch dieser Verschluss ist nicht für flüssigen Stuhl oder sonstige flüssige Anflutungen geeignet.

Eine Verwendung derartiger Stomakappen, wie sie nach Irrigation beim Colostoma eingesetzt werden, und damit eine zeitlich begrenzte "Befreiung" von einem Stomabeutel darstellen, sind für einen Ileo- oder Urostomaträger aber aus technischen Gründen nicht möglich. Bei einem Ileo- oder Urostoma fallen große Flüssigkeitsmengen an, für die ein herkömmlicher Colostomaverschluss weder geeignet noch ausgelegt ist. Hierfür werden bisher nur die bekannten Stomabeutel verwendet. Die betroffenen Ileo- oder Urostomaträger müssen derzeit ihr Leben mit einem Stomabeutel arrangieren. Betroffen sind über die Maßen junge Patienten, die an entzündlichen Darmerkrankungen leiden.

Aufgabe der Erfindung ist es insbesondere eine kostengünstige und leicht zu handhabende Vorrichtung zu schaffen, die es einem Betroffenen mit Ileostoma- oder Urostomaversorgung erlaubt, für eine gewisse Zeit ohne die notwendige Beutelversorgung auszukommen.

Die Erfindung stellt eine Vorrichtung für einen temporären Ileo- oder Urostomaverschluss nach dem Gegenstand des unabhängigen Anspruches 1 bereit.

Bevorzugte Ausführungsbeispiele der Erfindung ergeben sich aus den abhängigen Ansprüchen, der nachfolgenden Beschreibung und der beigefügten Zeichnung.

### KURZBESCHREIBUNG DER ZEICHNUNG

Weitere Ausführungsformen der Erfindung werden nun beispielhaft und unter Bezugnahme auf die angefügte Zeichnung beschrieben. In der Zeichnung zeigen schematisch:
Fig. 1 ein endständiges Ileostoma;
Fig. 2 ein doppelläufiges Ileostoma;
Fig. 3 ein Colon-Conduit;
Fig. 4 ein Ileum-Conduit;
Fig. 5 eine schematische Schnittdarstellung der erfindungsgemäßen Anordnung mit einer Verschlusskappe und einer Abdeckplatte auf einer Basisplatte;
Fig. 6 eine schematische Darstellung einer erfindungsgemäßen Vorrichtung von oben;
Fig. 7 eine schematische Schnittdarstellung einer erfindungsgemäßen Vorrichtung mit einem äußeren und einem inneren Folienbehälter;
Fig. 8 eine schematische Schnittdarstellung einer erfindungsgemäßen Vorrichtung mit der Abdeckplatte auf der Basisplatte mit einer Einführhilfe;
Fig. 9 eine schematische Schnittdarstellung einer erfindungsgemäßen Vorrichtung mit der Abdeckplatte auf der Basisplatte mit einem Ventilmechanismus;
Fig. 10 eine schematische Schnittdarstellung einer erfindungsgemäßen Vorrichtung mit der Abdeckplatte auf der Basisplatte mit einem wahlweise füllbarem Ballon;
Fig. 11 eine schematische Schnittdarstellung einer erfindungsgemäßen Vorrichtung mit der Abdeckplatte auf der Basisplatte mit einem Schirm;
Fig. 12 eine schematische Schnittdarstellung einer erfindungsgemäßen Vorrichtung mit der Abdeckplatte auf der Basisplatte mit einem geteilten Zylinder;
Fig. 13 eine schematische Darstellung des Zylinders aus Fig. 12; und
Fig. 14 eine schematische Darstellung eines alternativen Ausführungsbeispiels einer Vorrichtung für einen temporären Ileo- oder Urostomaverschluss.

Die Figuren sind nicht unbedingt maßstabsgetreu, einige Teile sind nur symbolisch dargestellt, Beachtung fand das Prinzip der Erfindung.

### DETAILLIERTE BESCHREIBUNG DER FIGUREN

Vor einer Beschreibung von Ausführungsbeispielen der erfindungsgemäßen Vorrichtung folgen zum besseren Verständnis einige Darstellungen verschiedener Arten von Stomata.

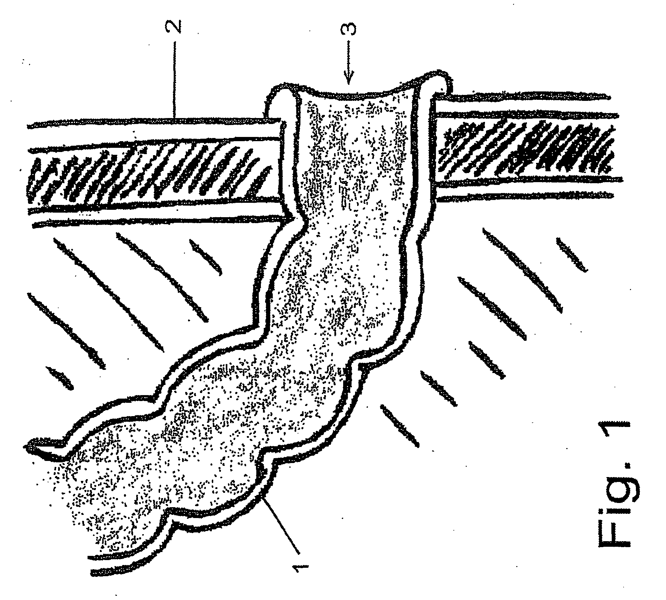

Fig. 1 zeigt ein endständiges Ileostoma mit dem zuführenden Darm 1, der nach einem Ausführungsbeispiel durch die Bauchdecke 2 einer Person geführt ist, und dort ein Stoma 3 bildet.

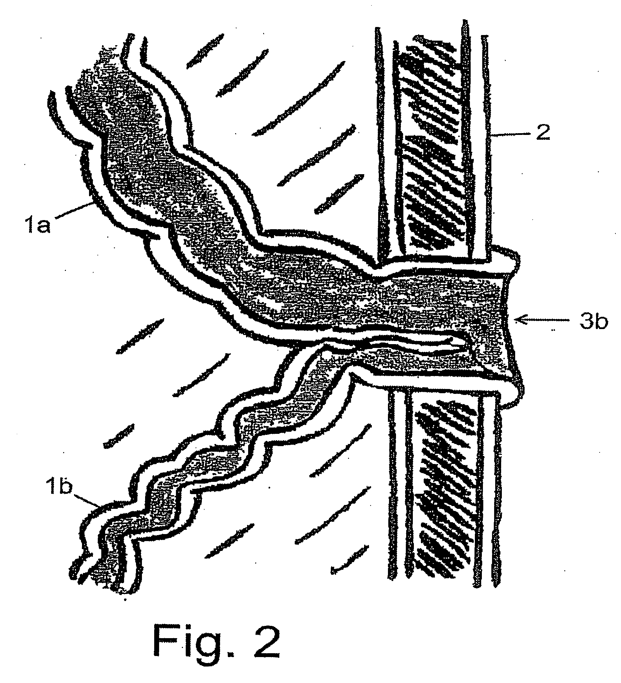

Fig. 2 zeigt nach einem weiteren Ausführungsbeispiel ein doppelläufiges Ileostoma mit einem zuführenden Darmteil 1 a, einem abführenden Darmteil 1 b und dem Stoma 3 an der Bauchdecke 2.

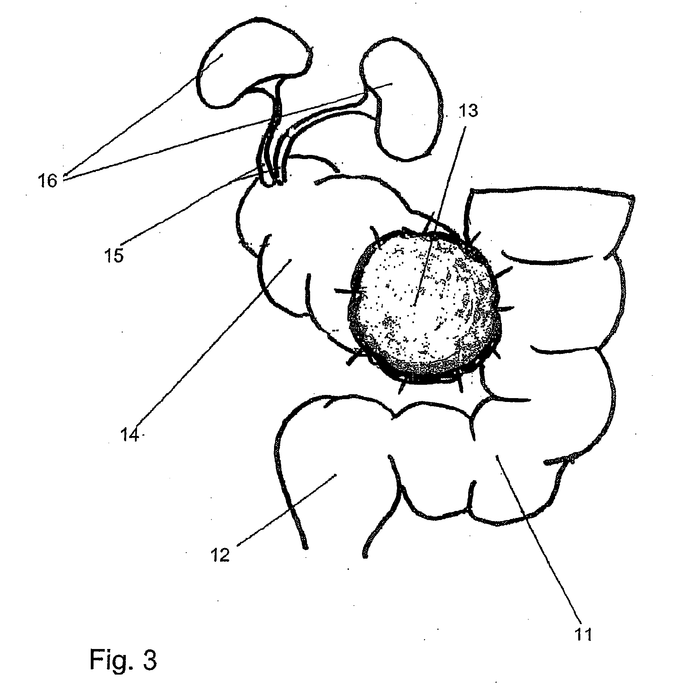

Fig. 3 zeigt nach einem weiteren Ausführungsbeispiel ein Colon-Conduit, bei dem Nieren 16 über implantierte Harnleiter 15 in ein ausgeschaltetes Dickdarm-Segment 14 entleert werden. Das Dickdarm-Segment 14 mündet in ein Stoma 13, das in die Bauchdecke (nicht dargestellt) eingenäht ist. Im Hintergrund sind ein Colon(Sigma) 11 und ein Rectum 12 zu sehen.

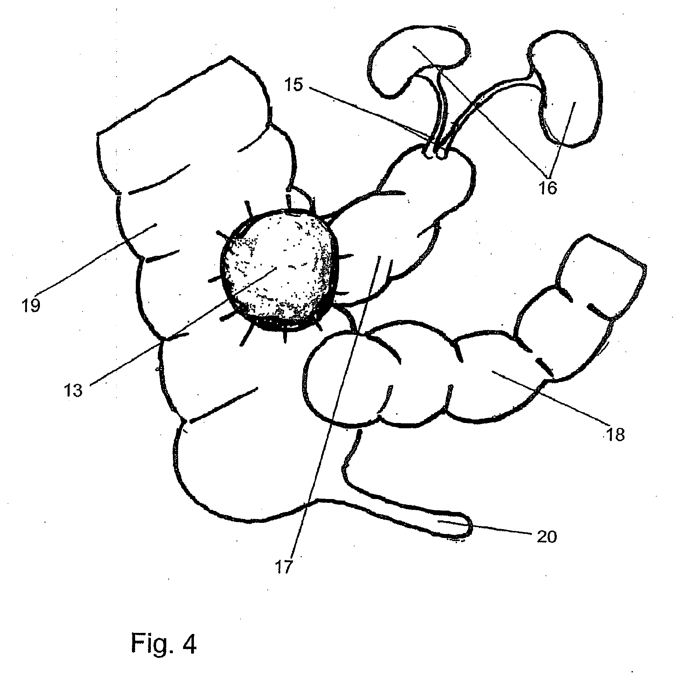

Fig. 4 zeigt nach einem weiteren Ausführungsbeispiel ein Ileum-Conduit, bei dem die Nieren 16 über die implantierten Harnleiter 15 in ein ausgeschaltetes Dünndarmsegment 17 entleert werden, wobei das Dünndarmsegment 17 in einem Stoma 13 mündet. Im Hintergrund sind ein Dünndarm 18, ein Appendix 20 und ein Colon ascendens 19 zu sehen.

Im nachfolgenden werden verschiedene Ausführungsbeispiele der erfindungsgemäßen Vorrichtung für einen temporären Ileo- oder Urostomaverschluss näher beschrieben. Dabei bezieht sich der Begriff "Stomaverschluss" auf einen Ileostomaverschluss bzw. einen Urostomaverschluss.

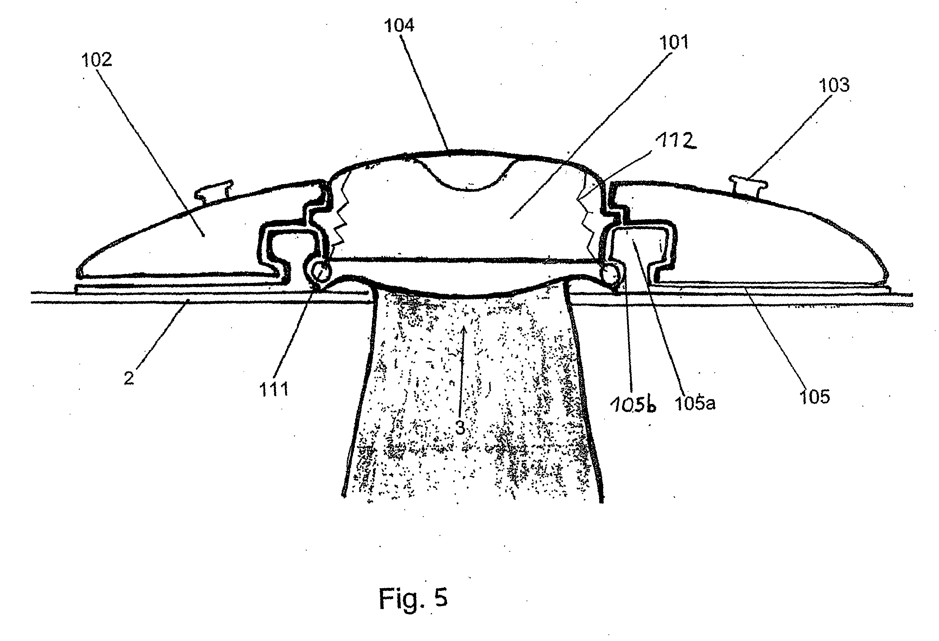

Fig. 5 zeigt ein erstes Ausführungsbeispiel einer erfindungsgemäßen Vorrichtung in einer schematischen Schnittdarstellung. Die erfinderische Vorrichtung soll einen aus dem Stand der Technik bekannten Beutel zur Aufnahme von aus dem Stoma austretenden Inhalt temporär ersetzen. Sie umfasst hierfür eine Basisplatte 105, die zur dauerhaften Anbringung in der Peripherie eines Stomas an der Außenseite einer Bauchdecke 2 ausgebildet ist. Des weiteren ist eine Abdeckung lösbar mit der Basisplatte 105 zum Bilden eines temporären Stomaverschlusses verbunden und in der Art einer halbschalenförmigen Verschlusskappe 101 so geformt ist, dass sie ein Aufnahmevolumen für die Aufnahme wenigstens eines Behältnisses in Form einer zusammengefalteten Behälterfolie 112, die in Verbindung mit den folgenden Figuren näher beschrieben wird, für aus dem Stoma 3 austretendes Fluid bildet. Wie man in Fig. 5 erkennt, ist das Behältnis an seinem offenen Ende lösbar mit der Basisplatte 105 gekoppelt. Die Verschlusskappe 101 kann aber unabhängig vom Behältnis von der Basisplatte gelöst werden. Die Basisplatte 105 ist mit einer umlaufenden Aussparung 105b versehen, an die das Behältnis an seinem offenen Ende eingehängt werden kann.

Auf der Basisplatte 105 mit einem umfänglichen Rastring 105a, die auf die gereinigte und entfettete Bauchdecke 2 aufgebracht ist, ist eine flache Abdeckplatte 102 befestigt. Diese Abdeckplatte 102 kann als wieder verwendbare Einheit ausgelegt sein und mit einem Antigeruchsmittel beschichtet werden. Die Beschichtung kann mit Metalloxiden, die als gute Katalysatoren bekannt sind, erfolgen. Diese können in Form nanoskaliger Teilchen in ein Sol-Gel Netzwerk eingebaut und auf der Abdeckplatte 102 aufgebracht werden. Zusätzlich kann eine Beschichtung, die schmutzabweisend wirken soll (Lotuseffekt), verwendet werden. Variationen solcher Beschichtungen finden bereits als "Antikratzlack" in der Autoindustrie Verwendung. In die Abdeckplatte kann in einem Ausführungsbeispiel eine elektronische Empfangseinheit für einen Füllstand-Sensor untergebracht werden. Die Lokalisation der Empfangseinheit ist so gestaltet, dass ein mechanischer oder sonstiger elektronischer Kontakt mit dem Sensor, der den Füllungszustand eines ausgeleiteten Stomas/Urostomas anzeigt, möglich ist. Die Abdeckplatte 102 besitzt in der Mitte eine zentrale Öffnung, um die halbschalenförmige Verschlusskappe 101 einzusetzen. Die ventrale Fläche der Abdeckplatte 102 kann einen Rastring 103 aufweisen. Nach Entfernen der Verschlusskappe 101 kann so wahlweise ein normaler Stomabeutel befestigt werden. Bevorzugt sind die Abdeckplatte und die Verschlusskappe aus einem nicht-deformierbaren oder nur geringfügig deformierbaren Material, beispielsweise Kunststoff, gebildet. In einem nichterfindungsgemäßen Beispiel bilden die Abdeckplatte und die Verschlusskappe eine integrale Einheit.

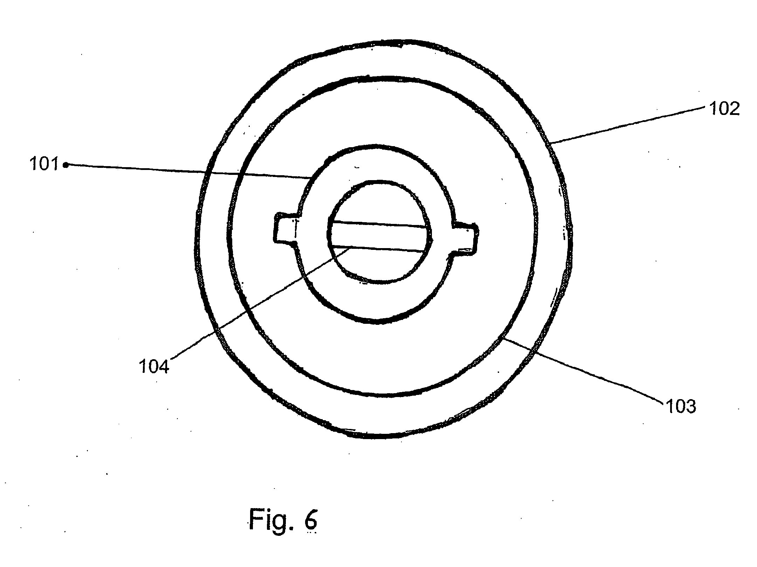

Fig. 6 zeigt ein Ausführungsbeispiel einer erfindungsgemäßen Vorrichtung mit Abdeckplatte 102 in der Draufsicht. Die auswechselbare, innen liegende Verschlusskappe 101 kann als Beutelersatz dienen. Die Verschlusskappe 101 ist beispielsweise mittels eines bajonettartigen Verschlusses mit einer Senk- und Drehbewegung in der Abdeckplatte 102 angebracht. Dazu befindet sich an der Verschlusskappe ein Eingriff 104. In einer alternativen, nichterfindungsgemäßen Ausführungsform können Abdeckplatte und Verschlusskappe in einem Stück ausgebildet sein.

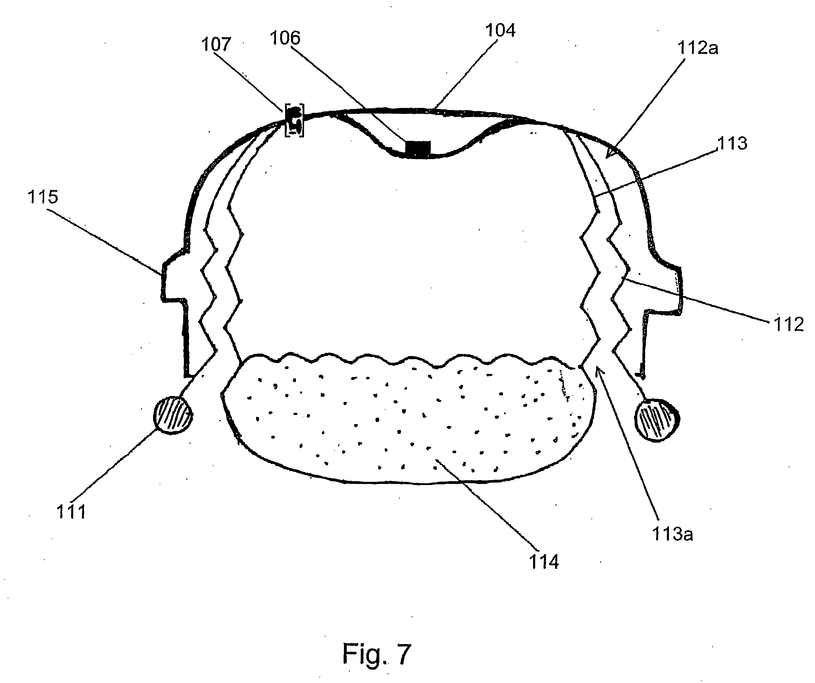

Nach Fig. 7 ist die Verschlusskappe 101 nach einem Ausführungsbeispiel der erfindungsgemäßen Abdeckung als Plastikhalbschale ausgebildet sein, in deren Inneren eine ziehharmonikaartig gefaltete Behälterfolie 112 aufgenommen ist. Die Behälterfolie 112 bildet einen ersten Folienbehälter 112a und kann auch die einzige Behälterfolie in einer Variante des Ausführungsbeispiels sein. Dieser (erste) Folienbehälter 112a entfaltet sich nach dem Lösen der Verschlusskappe 101 von der (nicht gezeigten) Basisplatte 105 und kann auf diese Weise nach Entfernen der Abdeckung den sich aus dem Darm entleerenden flüssigen oder semiflüssigen Darminhalt aufnehmen. Der dem Stoma 3 zugewandte Eingang des ersten Folienbehälteres 112a besitzt einen kreisrunden, umlaufenden Wulstring 111, der am Stomaausgang in Hautniveau in einer Aussparung an der Basisplatte 105 oder der Abdeckplatte 102 lösbar anliegt und abdichtet. In einer Ausführungsform ist der erste Folienbehälter ganz oder zum Teil aus einem metallenen Material hergestellt. Nach Füllung des Folienbehälteres 112a mit dem Darminhalt soll durch eine torquierende Bewegung an der Verschlusskappe erreicht werden, dass sich das offene in der Aussparung 105b der Basisplatte fixierte Ende des sich ausgedehnten Folienbehälteres durch die Drehbewegung verschließt und der Inhalt nicht ausfließen kann. Nach Füllung des Folienbehälteres bewirkt die torquierende Bewegung aufgrund der kurzzeitigen Befestigung des Wulstrings 111 an der Basisplatte 105 eine Torsion des Folienbehälteres. Diese Befestigung bewirkt bei einer Drehbewegung des Schlauches, dass sich der metallene Anteil des Schlauches torquierend verschließt. Sodann kann der gefüllte Folienbehälter von der Basisplatte entfernt werden. Alternativ oder zusätzlich kann zur Sicherung des Inhalts eine der im Handel üblichen Einmalklammern aufgesetzt werden.

Die Verschlusskappe kann einen Filter 107, insbesondere einen Aktivkohlefilter, umfassen, der die bei der Verdauung entstehenden Gase geruchsneutral entweichen lässt.

In einer weiteren Ausführungsform der Erfindung ist in der Verschlusskappe 101 eine zweite gefaltete Behälterfolie 113 angeordnet, die, z. B. durch kleine Löcher, für Flüssigkeit durchlässig ist. Diese zweite Behälterfolie kann an einem Ende an der Plastikhalbschale befestigt sein und im ersten Folienbehälter der ersten Behälterfolie 112 aufgenommen werden. Sie bewegt sich beim Lösen der Verschlusskappe 101 und anschließendem Entfalten der Folienschläuche wie eine Puppe in der Puppe. In einer Ausführungsform ist die Behälterfolie 113 etwas kürzer als der äußere Folienbehälter. Die Schläuche sind in Ihrer grundsätzlichen Form nicht beschränkt und können andere als runde Querschnitte haben. Vor dem Lösen der Verschlusskappe 101 aus der Abdeckplatte bildet die Behälterfolie 113 eine gefaltete Folieneinlage. Die Behälterfolie 113 bildet einen zweiten Folienbehälter 113a und enthält einen Quellstoff 114, vorzugsweise aus organischem Material, der sich bei geringem eigenem Volumen nach Kontakt mit Flüssigkeit ausdehnt und ein mehrfaches an Volumen aufnehmen kann. Solche Quellmittel werden auch als Superabsorber bezeichnet und sind von Inkontinenzartikeln bekannt. Dabei kann sich die perforierte Behälterfolie mit dem Quellmittel 114 wie ein abdichtender Pfropfen im Stomaausgang dehnen und damit weiter Flüssigkeit aufnehmen. Alternativ zum zweiten Folienbehälter 113a kann der erste Folienbehälter 112a durch eine flüssigkeitsdurchlässige und/oder elastische Behälterfolie unterteilt sein die einen Bereich mit Quellmittel 114 abtrennt.

Diese Maßnahmen bewirken zum einen, dass der Stomaausgang abgedichtet wird, zum anderen wird durch die Flüssigkeitsaufnahme der Druck auf die Verschlusskappe verringert und damit die Dichtigkeit erhöht. Um die Notwendigkeit eines Wechsels anzuzeigen, wird der Füllstand des Folieninhalts über Druck oder pH-Wert oder Leitwertmessung des Quellmittels 114 durch einen Sensor 106 erfasst. Weitere Möglichkeiten sind Volumenmessungen, Dehnungsmessstreifen der Foliendehnung bzw. - spannung. Der Messwert kann über Funktechnik wie z.B. RFID, ZIGBEE oder WLAN an entsprechende Empfänger weitergegeben und dort mechanisch (Wecker, Vibrator) oder optisch (LED-Ampel) dem Träger angezeigt werden. Das Signal kann abhängig von der Dringlichkeit unterschiedlich gestaltet sein. Zur Befestigung in der Abdeckplatte kann die Verschlusskappe einen Vorsprung 115 umfassen.

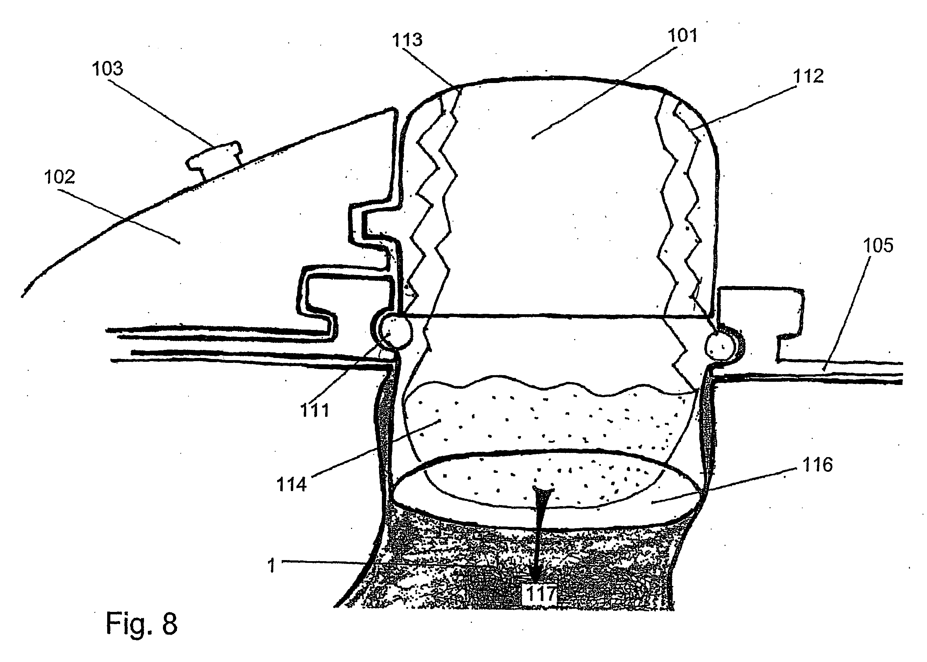

In einer Ausführungsform, dargestellt in Fig. 8, umfasst die Erfindung eine Einführhilfe im zuführenden Darm 1 a. Diese Einführhilfe umfasst ein Rohr 116, z. B. ein weiches Silikonrohr in Form eines Zylinders, welches mit einem Ende an der Basisplatte befestigt ist. Dieses Rohr dient als Führung für den inneren Folienbehälter mit dem organischen Quellmittel 114. Die innere Behälterfolie 113 mit dem Quellmittel 114 bewegt sich durch das Rohr 116 in den Darm hinein. Die Richtung wird durch einen Pfeil 117 angezeigt. Das Rohr soll verhindern, dass sich der Folienbehälter vor dem Stoma staut und durch die Volumenzunahme die Verschlusskappe 101 mit der Basisplatte 105 angehoben wird, was zu einer Undichtigkeit am Stomaausgang führen könnte.

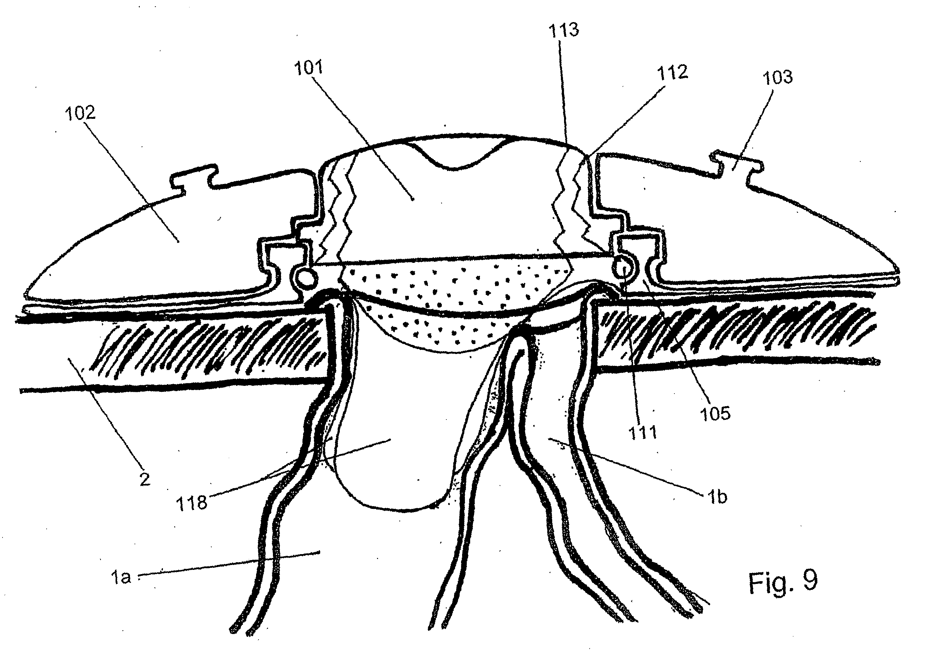

Fig. 9 zeigt eine weitere Ausführungsform mit einem ringförmigen weichen Kunststoffsegel 118, welches sich ähnlich der Bauhinschen Klappe am Übergang Ileum terminale und Caecum in das Stoma hineinwölbt. Diese Klappe ist so geformt, dass sie einen recht effektiven Verschluss in retrograder Richtung bei einem Wechsel bewirkt. Bei Dehnung des Folienbehälteres mit dem Quellmittel 114 wird das Kunststoffsegel 118 passiv in den Darm hinein aufgeweitet. Nach Entfernen des gefüllten Folienbehälteres fällt das weiche Kunststoffsegel wie eine Schleimhautfalte zusammen und hemmt den unkontrollierten Austritt von Flüssigkeit aus dem Stoma. Befestigt ist das ringförmige Kunststoffsegel an der Basisplatte 105 unmittelbar am Stoma.

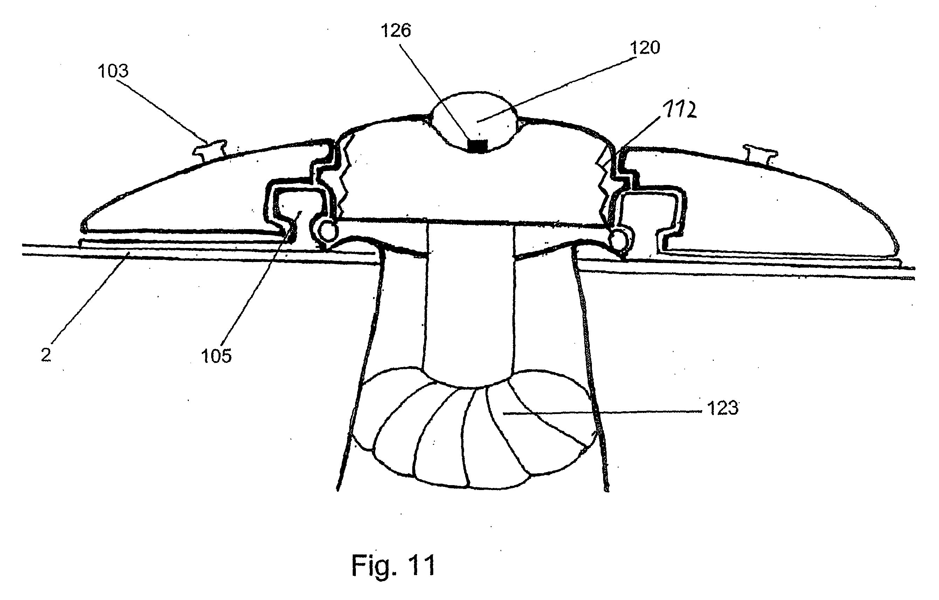

In einer alternativen Ausführungsform, wie sie in Fig. 11 dargestellt ist, umfasst die Vorrichtung ein Verbindungsstück, z.B. ein Rohr 120, vorzugsweise aus Silikon, welches mit einem Ende an der Innenseite der Verschlusskappe mittig befestigt ist. An dem anderen Ende des Rohres - im Darmlumen befindlich - ist eine Verlängerung mit einem schirmartigen Verschluss 123 angebracht, der sich nach dem Einsetzen der Verschlusskappe wie ein gewölbter Schirm, ausgelöst durch den Flüssigkeitsdruck aus dem Darmlumen, entfaltet. Dabei schmiegt sich der Schirmrand an die Wand des Darmlumens und bewirkt einen gewissen Flüssigkeitsrückhalt. Mittels eines Sensors 126, der zu dem in der Verschlusskappe befestigten Rohr 120 Kontakt hat und den darauf ausgeübten Druck misst, kann so der Flüssigkeitsdruck im Darmlumen gemessen und mit den oben beschriebenen Anzeigen weitergeleitet werden. Flüssigkeit, die sich an dem Schirmrand außen vorbeidrängt, wird durch ein Quellmittel, ähnlich wie in dem Beispiel der Fig. 7 und 8, absorbiert. Nach Überschreiten eines gewissen Druckquotienten (volumenbedingt), der vorher in den Meßwerten des Sensors festgelegt wird, wird das in der Verschlusskappe aufgehängte Silikonrohr um seine Achse gedreht, z. B. um 180°, und der Folienschirm 123 schließt sich wie ein Regenschirm. Die vermehrt anfallende Flüssigkeit, die jetzt nachfliest, wird vom vorgenannten Quellstoff absorbiert. Die Verschlusskappe 101, in der wie in den vorgenannten Beispielen eine gefaltete Behälterfolie 112 untergebracht ist, wird mit dem Silikonrohr wird in oben beschriebener Weise entfernt. Der Abschluss zum verhindern vom Austritt von Flüssigkeit erfolgt durch die oben beschriebenen weichen Kunststoffsegel 118.

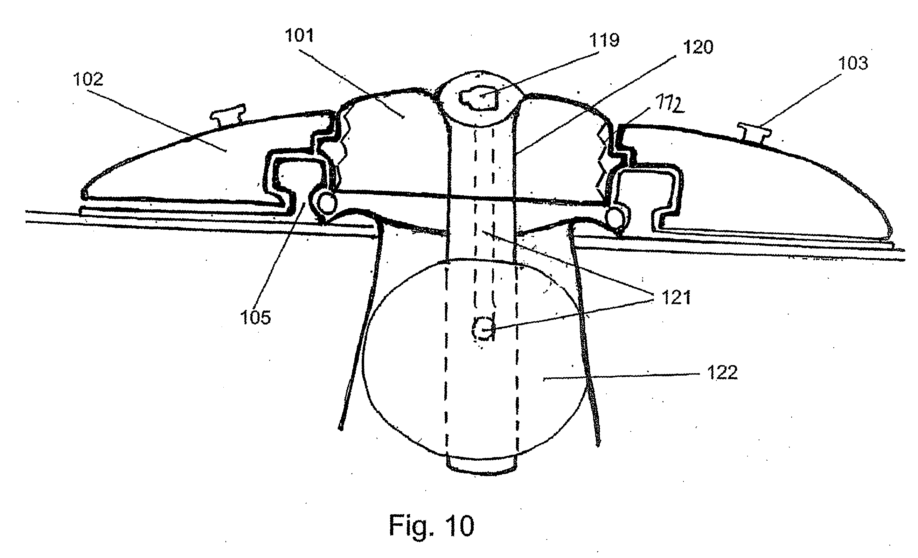

Gemäß Fig. 10 kann eine Ausführungsform der Erfindung, anstatt eines Folienschirms, der sich um ein Verlängerungsrohr entfaltet, einen aufblasbaren Ballon 122 umfassen, der an dem Rohr 120 angebracht wird und nach dem Prinzip der Ballonblockierung bei Harnkathetern, Intubationstubus etc. arbeitet. Dieser Ballon wird über einen Kanal 121 im Rohr 120 aufgeblasen und schmiegt sich als Ballonverschluss an die Innenseite des Darmlumens. Sich am Ballonrand vorbeidrängende Flüssigkeit, wird in dem Beispiel von Fig. 7 oder 8, von einem Quellmittel 114 absorbiert. Nach Überschreiten des Druckquotienten, der wie oben beschrieben durch einen Sensor 119, der zu dem in der Verschlusskappe befestigten Rohr 120 Kontakt hat und den darauf ausgeübten Druck misst, angezeigt wird, muss man vor dem Entfernen der Verschlusskappe 101 den Ballon 122 entlüften. Dies geschieht durch Öffnen eines Verschlusses, z. B. in Form einer Kappe oder eines Ventils (nicht dargestellt), an der Kappenaußenseite. Der Ballon fällt in sich zusammen, anfallende Flüssigkeit wird über das Quellmittel 114 absorbiert. Ballon und Verschlusskappe samt Behälterfolie 112 können mit dem Folieninhalt entfernt werden. Die Kunststoffsegel 118, die als Verschluss dienen, verhindern ein weiteres Austreten von Flüssigkeit.

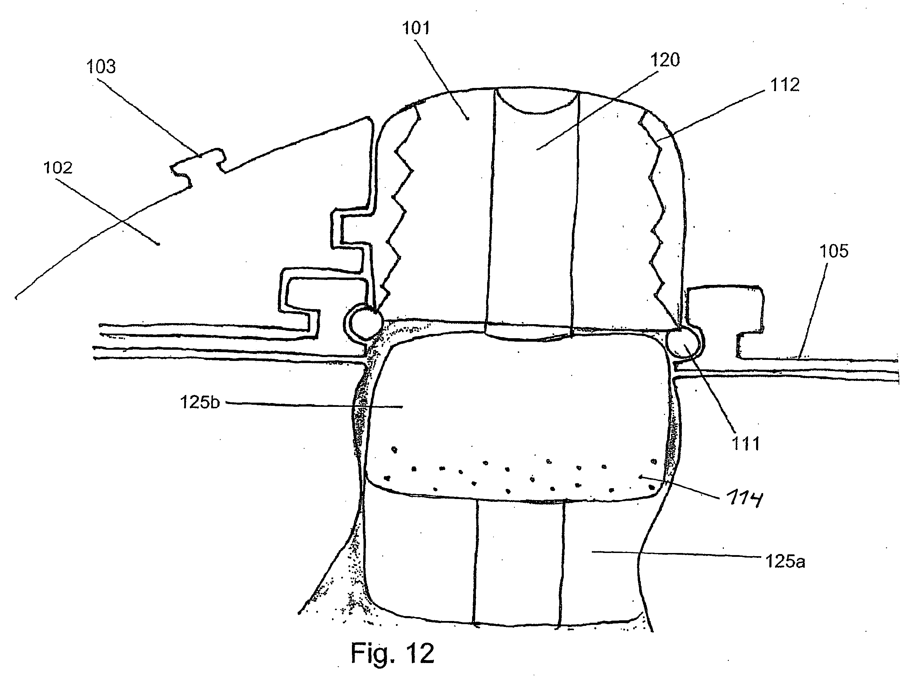

In einer weiteren Ausführungsform der Erfindung gemäß Fig. 12 ist ein Zylinder vorgesehen, der nach der gleichen Art wie oben beim Ballon beschrieben, in den zuführenden Darm über eine an der Verschlusskappe 101 angeordnete Verlängerung 120 eingeführt wird. Der Zylinder umfasst zwei Kammern. Die untere zum Darmlumen gerichtete Kammer 125a dient als Einführhilfe und ist wie der Ballon 122 in Fig. 10 aufblasbar. Die obere Kammer 125b dient der Aufnahme des Darminhaltes (ähnlich wie der innere Folienbehälter 113 in dem Beispiel der Fig. 7) und ist mit einem Quellmittel 114 gefüllt. Beim Entleeren wird die Verschlusskappe 101, in der wie in den vorgenannten Beispielen eine gefaltete Behälterfolie 112 untergebracht ist, mit dem Zylinder in oben beschriebener Weise entfernt, so dass der Zylinder samt Quellmittel in der Behälterfolie entsorgt werden kann.

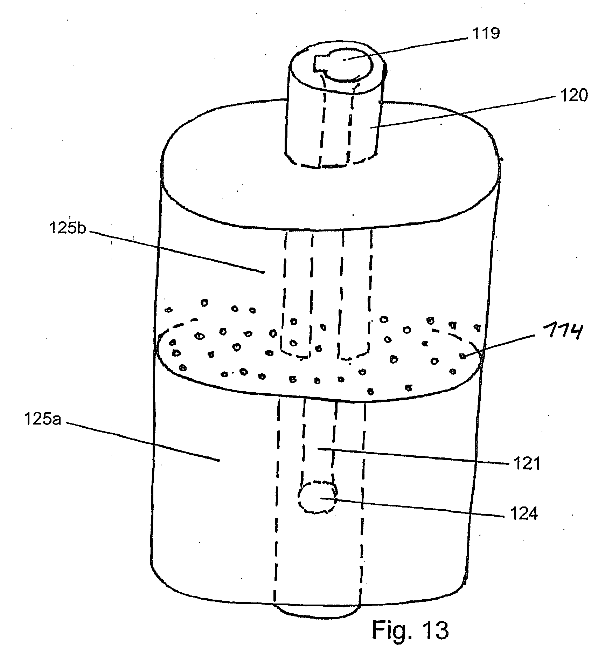

In Fig. 13 ist das zylindrische Behältnis im Detail dargestellt. Wie in der Ausführungsform mit Ballon (vgl. Fig. 10) ist der Zylinder über ein Rohr 120 an der Verschlusskappe befestigt, durch welches ein Kanal zur Be- und Entlüftung geführt ist. Das Rohr 120 weist im Bereich der unteren Kammer 125a eine Öffnung 124 zum dem Kanal auf. Am oberen Ende des Rohrs 120 befindet sich eine Be- und Entlüftungsöffnung 119. Durch die zylinderförmige Gestaltung wird die Einführung in das Stoma deutlich erleichtert.

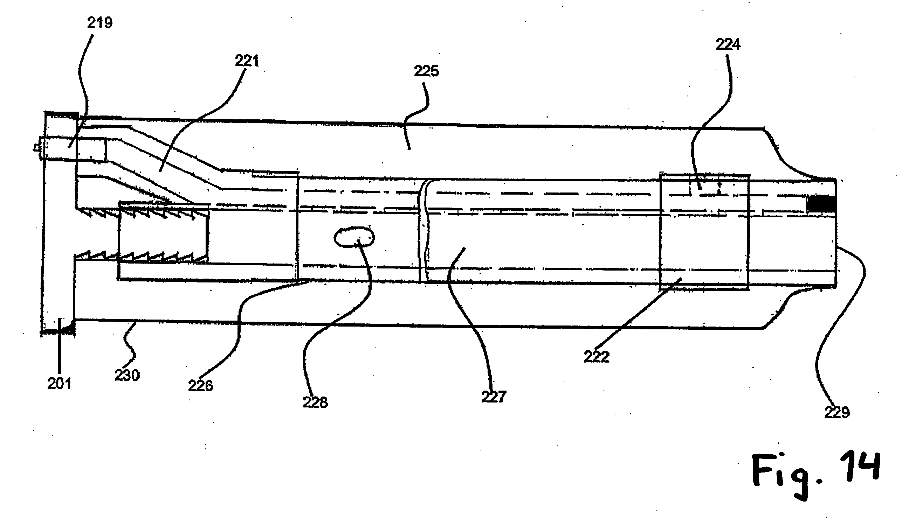

Fig. 14 zeigt ein alternatives Ausführungsbeispiel einer Vorrichtung für einen temporären Ileo- oder Urostomaverschluss. Die Länge dieser Verschlusseinheit ist dem jeweiligen Einsatzgebiet, d.h. ob Ileo- oder Urostoma, angepasst. Die Vorrichtung umfasst ein in das Stoma hineinragendes einseitig offenes Rohrelement, das als Einführhilfe dient und an dessen zum Darmlumen hin gerichteten Endabschnitt eine aufblasbares Volumen vorgesehen ist, derart, dass das Rohrelement im Darm fixiert und gleichzeitig durch das aufblasbares Volumen das Stoma abdichtet wird. Ferner umfasst die Vorrichtung eine Kammer, die um das Rohr herum angeordnet und zur Aufnahme des Darminhaltes ausgebildet ist.

In einer bevorzugten Ausführungsform wird als Rohrelement ein modifizierter Katheter 226 z.B. aus weichem Silikon verwendet, der an der Spitze mit einem aufblasbaren Ballon 222 aus Silikon ausgestattet ist. Solche sog. Ballon-Katheter werden üblicherweise zur transurethralen Blasendrainage verwendet. Ein Ballon-Katheter wird für gewöhnlich aus weichem Silikon oder Latex gefertigt und ist in Nähe der Spitze mit einem aufblasbaren kleinen Ballon aus Silikon versehen, der, wenn er aufgeblasen ist, den Ballon-Katheter normalerweise in der Harnblase verblockt.

Der im vorliegenden Ausführungsbeispiel verwendete Katheter 226 weist einen Durchmesser von ca. 28 - 30 Charriere (ungefähr 9 - 10 mm) auf. Ein axial verlaufender Hauptkanal 227 des Katheters 226 verbindet eine Eingangsöffnung 229 mit einer Öffnung 228 zu einer Kammer 225, die als elastischer Folienbehälter 230 in Zylinderform ausgebildet ist, und die um den Katheter 226 herum angeordnet und zur Aufnahme des Darminhaltes ausgebildet ist. Der Katheter 226 wird an einem Ende an der Innenseite einer Verschlusskappe 201 gehalten. Die Eingangsöffnung 229 am anderen Ende des Katheters 226 mündet in das Darmlumen. Der Rand der Eingangsöffnung 229 ist abgerundet und weich. Die Kammer 225 ist um den Katheter 226 angeordnet und wird von der Innenseite der Verschlusskappe 201 am Stoma und durch einen elastischen Behälter zu den Seiten hin begrenzt. In dem gezeigten Ausführungsbeispiel wir der Behälter durch einen elastischen Folienbehälter 230 in Zylinderform gebildet. Dieser Stellt die Abgrenzung zum Innenlumen des Darms dar und enthält ein Quellmittel (nicht gezeigt) wie es bereits im Zusammenhang mit anderen Ausführungsbeispielen beschrieben wurde. In einem besonderen Ausführungsbeispiel enthält auch der Hauptkanal 227 ein Quellmittel.

Der elastische Folienbehälter 230 ist bevorzugt aus einem Schlauch aus dünnem Silikon oder Latex gefertigt, der den Katheter 226 und die Kammer 225 ummantelt. Der elastische Folienbehälter 230 wird distal des Ballons 222 ringförmig in Nähe der Eingangsöffnung 229 mit dem Katheter 226 verbunden, beispielsweise verklebt oder verschweißt, zieht sich über den aufblasbaren Ballon und wird am proximalen Ende mit der Innenseite der Verschlusskappe 201 z.B. mit einem Spreng- oder Pressring verbunden. Dazu wird in die Innenseite der Verschlusskappe 201 eine Vertiefung oder Rille zirkulär eingefräst, in die der proximale elastische Folienbehälter sicher durch Klemmverbindung verankert werden kann. Der Folienbehälter 230 bildet somit ein zu den Seiten geschlossenes System. Der Folienbehälter 230 umhüllt dabei Den Katheter und am distalen Ende den aufblasbaren Ballon 222 und ist mit dem Katheter 226 bzw. dem Hauptkanal 229 verschweisst und am proximalen Ende in der Verschlußkappe 201 verankert. Der Folienbehälter 230 ist so dimensioniert, dass er vor dem Einsetzen in das Stoma 3 locker um den Katheter 226 gelegt wird, und ist ausgestaltet, um sich beim Füllen der Kammer 225 mit Flüssigkeit aus dem Darm zylinderförmig um den eingeführten Katheter 226 bis zur Darminnenwand auszudehnen. Der maximal erreichbare Durchmesser des Folienbehälteres 230 im Darm wird von der Darmwandelastizität mitbestimmt.

In diesem Ausführungsbeispiel ist die Öffnung 228 oval und in Nähe der Verschlusskappe 201 angebracht. Die Öffnung 228 im Hauptkanal 227 ist in einem Ausführungsbeispiel mit einer Membrane (nicht dargestellt) versehen, die als Einwegöffnung wie ein Rückschlagventil nur Flüssigkeit aus dem Hauptkanal 227 in die Kammer 225 fließen lässt. Die Membrane soll bei Veränderung einer Körperlage des Trägers die Öffnung 228 zwischen der Kammer 225 und dem Hauptkanal 227 verschließen und einen Rückfluss aus der Kammer 225 in den Hauptkanal 227 verhindern.

Im Inneren des Katheters 226 verbindet ein Ballonkanal 221 einen Anschluss 219 an der Verschlusskappe 201 mit dem Ballon 222. Der Ballonkanal 221 mündet in den Ballon 222 in einer Öffnung 224 in der Wand des Ballonkanals 221. Über den Anschluss 219 und den Ballonkanal 221 kann der Ballon 222 mit Hilfe einer Injektionsvorrichtung, beispielsweise eine Injektionsspritze, mit Luft oder einer Flüssigkeit gefüllt werden, beispielsweise mit einem Volumen von ca. 5-10 ml. Auf diese Weise wird der Katheter 226 im Darm gehalten und dichtet ihn an der Stelle des sich ausdehnenden Ballons 222 zum Stoma 3 hin ab. Der Anschluss 219 ist in manchen Ausführungsbeispielen mit einem Rückschlagventil ausgestattet, das in besonderen Ausführungsbeispielen entsperrbar ausgeführt ist. Das Rückschlagventil hält den Ballon 222 gefüllt, solang der Katheter 226 im Darm verbleiben soll. Wird das Rückschlagventil am Anschluss 219 entsperrt, z.B. durch Eindrücken eines in Form eines Druckknopfes ausgeführten Werkzeuges, wird die Füllung aus dem Ballon 222 entlassen und der Katheter wird nicht mehr durch den Ballon 222 gehalten. Der Ballonkanal 221 ist in Nähe der Eingangsöffnung 229 verschlossen.

Zur Verwendung führt der Träger diese erfindungsgemäße Vorrichtung über den Katheter 226 in das Stoma ein. Wenn der Katheter 226 weit genug eingesetzt ist, setzt der Träger die Verschlusskappe 201 in die Abdeckplatte 102 ein und verriegelt sie dort. Danach bläst der Träger den Ballon 222 z.B. mit einer Spritze auf. Der aufgeblasene Ballon 222 hält den Katheter 226 im Darm und verhindert, dass Darmflüssigkeit außen am Katheter 226 vorbeifließt.

Solang der Katheter 226 auf diese Weise eingesetzt ist, gelangt Darmflüssigkeit durch die Eingangsöffnung 229 aus dem Darm in den Hauptkanal 227 des Katheters 226. Die Darmflüssigkeit fließt dann durch die Öffnung 228 aus dem Hauptkanal 227 in die Kammer 225, welche vom Folienbehälter 230 umgeben ist. Ein Quellmittel im Folienbehälter 230 nimmt die Darmflüssigkeit auf und der Folienbehälter 230 weitet sich. In einem Beispiel bindet das Quellmittel die Darmflüssigkeit in der Kammer 225 in geleeartiger Form. Einmal aufgenommene Darmflüssigkeit bleibt so in der Kammer 225 dauerhaft gebunden, was später auch eine Entsorgung vereinfacht. Das Quellmittel unterstützt außerdem aufgrund eines Kapillareffekts die Aufnahme von Darmflüssigkeit in der Kammer 225 und die Ausdehnung des Folienbehälters 230 zusätzlich, wodurch in der Kammer 225 mehr Flüssigkeit aufgenommen werden kann. Außerdem wird durch den Kapillareffekt des Quellmittels schon bei einem geringen Darminnendruck Darmflüssigkeit in der Kammer 225 aufgenommen.

In einem weiteren Ausführungsbeispiel ist in der Verschlusskappe 201 ein Spülanschluss (nicht dargestellt) angeordnet, der ebenfalls mit einem einfachen Rückschlagventil versehen ist und von dem sich ein Spülkanal in Richtung Hauptkanal 227 erstreckt. Der Spülkanal mündet zwischen der Verschlusskappe 201 und der Öffnung 228 in den Hauptkanal 227. Über den Spülkanal wird eine Spülflüssigkeit, beispielsweise Wasser oder eine NaCl-Lösung, mit einer Spritze bei Bedarf in den Hauptkanal 227 gespritzt. Die Spülflüssigkeit soll den Hauptkanal 227 durchlässig halten, insbesondere soll sie eventuelle Verklebungen durch Fasermaterial, Eiweißflocken etc. beseitigen. Diese festen Bestandteile werden mit der eingebrachten NaCl-Lösung verdünnt und ins Darmlumen gespült. In einem besonderen Ausführungsbeispiel wird die Öffnung 228 verschlossen, bevor die Spülflüssigkeit in den Hauptkanal 227 gespritzt wird. Dazu verschließt beispielsweise ein Schieber die Öffnung 228 wenn die Spritze in den Spülanschluss eingeführt wird. Auf diese Weise wird vermieden, dass die Spülflüssigkeit während des Spülens durch die Öffnung 228 in die Kammer 225 entweicht.

Bei einem weiteren Ausführungsbeispiel ist ein Sensor zum Messen des Füllstands am Katheter 226 oder im Inneren der Kammer 225 vorgesehen, der ein Signal an einen entsprechenden Empfänger z.B. in der Abdeckplatte 102, bevor der Folienbehälter vollständig gefüllt ist, sendet. Der Sensor ist beispielsweise ein Drucksensor, der durch das aufsteigende Quellmittel ausgelöst wird, oder ein anderer der weiter oben erwähnten Sensoren. Der Träger empfängt dann wie oben beschrieben den Messwert bzw. eine Empfehlung zum Wechsel der Verschlusskappe 201 mit dem Katheter 226 und dem Folienbehälter 230 samt Inhalt.

Zum Entfernen der Verschlusskappe 201 mit anhängendem Folienbehälter 230 samt Inhalt, wird zunächst der Ballon 222 über den Anschluss 219 entlüftet. Dazu wird gegebenenfalls das Rückschlagventil mittels einer Spritze entsperrt, bzw. durch Einsetzen des oben erwähnten Druckknopfes, der das Ventil entsperrt. In einem Ausführungsbeispiel löst der Träger die Verschlusskappe 201 aus der Abdeckplatte 102 und zieht den Folienbehälter 230 heraus. Das Quellmittel im Folienbehälter hält dabei die Flüssigkeit im Inneren des Folienbehälteres 230. Eine Behälterfolie, wie die Behälterfolie 112 aus Fig. 7, die in der Verschlusskappe 101 untergebracht ist und in der Abdeckplatte 102 gehalten wird, kann zusätzlich vorgesehen sein und umhüllt den Folienbehälter 230 beim Herausziehen.

In einem anderen Ausführungsbeispiel wird eine solche Behälterfolie (nicht dargestellt) erst kurz vor der Entnahme des Katheters 226 angebracht. Dazu ist eine separate Behälterfolie als ziehharmonikaähnlich gefalteter Folienbehälter ausgebildet. Nachdem der Ballon 222 entlüftet wurde, wird die Behälterfolie mit einem offenen Ende auf den Rastring 103 der Abdeckplatte 102 geklemmt. Die Behälterfolie ist so ausgeführt, dass der Träger den Eingriff 104 an der Verschlusskappe 201 durch die Behälterfolie fassen kann. Dazu ist die Behälterfolie entweder dünn und nachgiebig ausgeführt oder sogar mit einem Loch versehen, durch das die Verschlusskappe 201 gefasst werden kann. Gegebenenfalls kann der Rand des Lochs rundum mit der Verschlusskappe verklebt werden, so dass keine Darmflüssigkeit entweicht. Der Träger löst den Verschluss der Verschlusskappe 201 über den Eingriff 104 und zieht den Katheter 226 mit dem Folienbehälter aus dem Darminneren. Die Behälterfolie umhüllt die Verschlusskappe 201 und den Folienbehälter 230 mit seinem gesamten Inhalt zur Gänze. Die Behälterfolie erlaubt so eine berührungsfreie und saubere Entsorgung.

Nach der Entfernung des Inhaltes ist das Stoma 3 kurzzeitig offen und nur mit der Abdeckplatte 102 versehen. Der Träger kann jetzt entweder eine weitere Verschlusskappe 201 mit einem weiteren Katheter 226 einsetzen oder einen Stomabeutel auf dem Rastring der Abdeckplatte 102 aufbringen.

Wie beim Wechsel eines Stomabeutels sollte beim Wechsel der Verschlusskappe 201 nicht viel flüssiger Darminhalt aus dem Stoma 3 austreten. Beim Wechsel von Stomabeuteln genügt normalerweise ein kurzes Aufnehmen der Flüssigkeit durch Abtupfen des Stomas 3 mit einer Kompresse.

Eine weitere nicht dargestellte Ausführungsform der Erfindung umfasst mindestens eine, bevorzugt zwei halbe Scheiben, die federnd gelagert sind und am oberen Rand der Basisplatte befestigt sind. Beim Einführen der Verschlusskappe in die Abdeckplatte weichen die beiden Halbscheiben ungefähr parallel zur Bauchdecke jeweils in die zugehörige Hälfte der Höhlung der Abdeckplatte zurück. Durch den einfachen Federmechanismus werden diese beiden Halbscheiben beim Entfernen der Verschlusskappe geschlossen und bilden über dem Stomaausgang eine Art Ventilsperre. Dies dient als zusätzliche Sicherung gegen den Austritt von Darminhalt beim Wechsel.

Die Ausführungsbeispiele der erfindungsgemäßen Vorrichtung stellen praktische Hilfsmittel für Patienten mit einem Ileostoma bzw. Urostoma bereit, bei dem die Harnleiter über ein Ileum-Conduit oder Colon-Conduit ausgeleitet werden. Es soll während eines limitierten Zeitraums den Verzicht auf einen herkömmlichen Stomabeutel ermöglichen. Ein künstlicher Darm- oder Blasenausgang beeinflusst stark das Sozial- wie auch das Intimleben der Betroffenen. Wegen der Tabuisierung der Themen Urin und Stuhl treten häufig Scham und Minderwertigkeitskomplexe auf. Die Erfindung stellt eine temporäre Abdeckung eines Stomas bereit, die so ausgestaltet ist, dass bei einem Wechsel der Abdeckung austretendes Fluid in einem Behältnis aufgenommen wird bzw. in das Darmlumen eingeführt dort bereits Fluid in ein Behältnis aufnimmt. Eine Erleichterung in der Bewältigung dieser einschneidenden Veränderungen wird mit den Ausführungsbeispielen der erfindungsgemäßen Vorrichtung erreicht und damit das Selbstwertgefühl gesteigert und die Lebensqualität und Mobilität der Betroffenen verbessert. Weiter schaffen die Ausführungsbeispiele der Erfindung eine kostengünstige und leicht zu handhabende Vorrichtung, die es einem Betroffenen z.B. mit Ileostoma/Urostomaversorgung erlaubt, für eine gewisse Zeit ohne die notwendige Beutelversorgung auszukommen. Ferner schaffen die Ausführungsbeispiele der Erfindung es, eine sichere Stomaversorgung zu gewährleisten, dem Stomaträger die "Geheimhaltungsstrategie über die Andersartigkeit" zu nehmen, den Betroffenen ein neues Selbstwertgefühl zu geben und ihn Gesellschaftsfähig zu machen.

## Patentansprüche

1. Vorrichtung für einen temporären Ileo- oder Urostomaverschluss, umfassend:
- eine Basisplatte (105) zur dauerhaften Anbringung in der Peripherie eines Stomas an der Außenseite der Körperoberfläche (2),
- wenigstens ein temporär angeordnetes Behältnis zur Aufnahme von aus dem Stoma (3) austretenden Inhalt, und
- eine Abdeckung, die lösbar mit der Basisplatte (105) verbunden ist, **dadurch gekennzeichnet, dass**
- die Abdeckung derart geformt ist, dass sie ein Aufnahmevolumen für die Aufnahme des Behältnisses bildet, wobei die Abdeckung aus einer Verschlusskappe (101) und einer Abdeckplatte (102) gebildet ist, welche die Verschlusskappe (101) in einer Öffnung der Abdeckplatte an die Basisplatte (105) über der Öffnung eines Stoma (3) koppelt,
- das Behältnis nach Art eines einseitig offenen Schlauches (112a) ausgebildet ist, der in der Verschlusskappe (101) angeordnet ist, mit dem geschlossenen Ende an der Verschlusskappe (101) befestigt ist und ausgestaltet ist, um sich beim Herausziehen der Verschlusskappe (101) aus der Abdeckplatte (102) zu entfalten und aus dem Stoma (3) austretenden Inhalt aufzunehmen.

2. Vorrichtung nach Anspruch 1, wobei die Basisplatte (105) eine Aussparung (105b) umfasst, in die das Behältnis an einem offenen Ende davon eingehängt werden kann.

3. Vorrichtung nach Anspruch 1 oder 2, wobei die Abdeckplatte (102) einen Rastring (103) umfasst, der die Öffnung der Abdeckplatte umgibt, um einen Stomabeutel in der Peripherie eines Stomas (3) flüssigkeitsundurchlässig und lösbar mit der Abdeckplatte zu verbinden.

4. Vorrichtung nach einem der vorstehenden Ansprüche, wobei der Schlauch (112a) aus einem verformbaren Material ausgebildet und unter Verformung im Aufnahmevolumen der Abdeckung untergebracht ist.

5. Vorrichtung nach einem der vorstehenden Ansprüche, wobei der Schlauch (112a) mindestens teilweise aus einer metallenen Behälterfolie (112) ausgebildet ist..

6. Vorrichtung nach einem der vorstehenden Ansprüche, wobei der Schlauch (112a) am offenen Ende einen Wulstring (111) umfasst, der ausgestaltet ist, um in einer umlaufenden Aussparung (105b) an der Basisplatte (105) und/oder der Abdeckplatte (102) aufgenommen zu werden und die Öffnung der Basisplatte nach außen abzudichten.

7. Vorrichtung nach einem der vorstehenden Ansprüche, wobei in dem Schlauch (112a) durch eine flüssigkeitsdurchlässige Wandung (113) ein Bereich zur Aufnahme von Flüssigkeit festgelegt ist, der als weiterer Schlauch (113a) und mindestens teilweise verformbar und/oder faltbar ausgestaltet ist.

8. Vorrichtung nach Anspruch 7, wobei der weitere Schlauch (113a) kürzer ist als der Schlauch (112a) und mit einem Ende an der Verschlusskappe befestigt ist.

9. Vorrichtung nach einem der vorstehenden Ansprüche, wobei in dem Schlauch (112a) und/oder in dem Bereich zur Aufnahme von Flüssigkeit ein Quellmittel (114) angeordnet ist.

10. Vorrichtung nach Anspruch 9, wobei die Wandung (113) ausgestaltet ist, um sich bei Aufnahme von Flüssigkeit durch Quellen des Quellmittels (114) in Richtung des Stomas (3) auszudehnen.

11. Vorrichtung nach einem der Ansprüche 9 bis 10, wobei die Vorrichtung eine Sensoreinheit (106) umfasst, die ausgestaltet ist, um aufgrund eines Sensorwertes die Notwendigkeit eines Wechsels anzuzeigen, wobei die Sensoreinheit (106) eingerichtet ist, um Druck, pH-Wert und/oder Leitwert des Quellmittels (114) zu erfassen, und eine Auswerteeinheit aufweist, um die Notwendigkeit eines Wechsels zu ermitteln.

12. Vorrichtung nach einem der der Ansprüche 9 bis 11, wobei an der Basisplatte (105) eine Einführhilfe (116) für die sich durch das Quellmittel (114) bei Flüssigkeitaufnahme ausdehnende Wandung (113) befestigt ist.

13. Vorrichtung nach einem der Ansprüche 9 bis 12, wobei an der Basisplatte (105) ein Ventil in Form eines Kunststoffsegels (118) befestigt ist, das ausgestaltet ist, um durch das Quellmittel (114) hinter der Wandung (113) bei Flüssigkeitsaufnahme geöffnet zu werden und das Stoma nach Entfernen der Verschlusskappe mit Folien- und Flüssigkeitsinhalt zu schließen.

14. Vorrichtung nach einem der vorstehenden Ansprüche, wobei die Abdeckung über ein Verbindungsstück (120) mit einem schirmartigen Element (123) verbunden ist, das sich in den Darm hineinerstreckt und ausgestaltet ist, um sich am der Abdeckung gegenüberliegenden Ende des Verbindungsstücks durch Darminnendruck oder durch Einwirken eines Trägers zu entfalten und Flüssigkeitsaustritt zu hemmen.

15. Vorrichtung nach einem der vorstehenden Ansprüche, wobei die Vorrichtung einen in das Stoma eingeführten aufblasbaren Ballon (122) umfasst, der ausgestaltet ist, um von außen be- und entlüftet zu werden, um das Stoma (3) wahlweise abzudichten.

16. Vorrichtung nach einem der vorstehenden Ansprüche, wobei an der Innenseite der Abdeckung (101, 102) ein in das Urostoma hineinragendes zylinderförmiges Behältnis vorgesehen ist, das eine zum Darmlumen hin gerichtete erste Kammer (125a), die aufblasbar ausgebildet ist, und eine an die erste Kammer (125a) angrenzende zweite Kammer (125b) aufweist, die ein Quellmittel 3(114) zur Aufnahme des aus dem Urostoma (3) austretenden Inhaltes enthält.

17. Vorrichtung nach einem der vorstehenden Ansprüche, wobei die Abdeckung und/oder die Basisplatte (102) mindestens teilweise mit einem Material beschichtet sind, das einem Anhaften von Schmutzpartikeln und/oder einer Geruchsbildung entgegenwirkt.

## Claims

1. Apparatus for a temporary ileostomy or urostomy cap, including:
- a baseplate (105) for durable attachment on the periphery of a stoma on the outside of the body (2),
- at least one temporary receptacle to collect the contents leaving the stoma (3),
and
- a cover, which is detachable from the baseplate (105), **characterized in that**
- the cover shaped in such a way that it forms a collection capacity for the receptacle, whereby the cover consists of a sealing cap (101) and a cover plate (102), where the sealing cap (101) connects to the baseplate in an opening of the cover plate (105) above the opening of a stoma (3),
- the receptacle is formed as a tube open on one side (112a), which is positioned in the sealing cap (101), where the closed end is mounted to the sealing cap (101) and it is designed to unfold when pulling the sealing cap (101) out from the cover plate (102) and to collect the content leaving the stoma (3).

2. Apparatus according to Claim 1, whereby the baseplate (105) includes an opening (105b) on which the receptacle can be mounted at an open end.

3. Apparatus according to Claim 1 or 2, whereby the cover plate (102) includes a flange ring (103) which surrounds the opening of the cover plate in order to impermeably and detachably connect an ostomy pouch on the periphery of a stoma (3) to the cover plate.

4. Apparatus according to one of the above Claims, whereby the tube (112a) is formed from a malleable material and, by deforming it, is stored in the capacity of the cover.

5. Apparatus according to one of the above Claims, whereby the tube (112a) is at least partially formed from a metallic container foil (112).

6. Device according to one of the above Claims, whereby the tube (112a) includes an o-ring on the open end (111), which is designed to be assimilated into a wrap-around recess (105b) on the baseplate (105) and/or the cover plate (102) and to seal the opening of the baseplate toward the outside.

7. Apparatus according to one of the above Claims, whereby an area for collecting fluid is defined by permeable walls (113) inside the tube (112a), which is designed as an additional tube (113a) and which is designed to be at least partially malleable and/or collapsible.

8. Apparatus according to Claim 7, whereby the additional tube (113a) is shorter than the tube (112a) and is attached to the sealing cap at the end along with the tube.

9. Apparatus according to one of the above Claims, whereby an expanding agent (114) is positioned in the tube (112a) and/or in the area for collecting fluid.

10. Apparatus according to Claim 9, whereby the walls (113) are designed to expand by swelling the expanding agent (114) toward the stoma (3) when collecting fluid.

11. Apparatus according to one of Claims 9 to 10, whereby the apparatus includes a sensor unit (106) which is designed to indicate that a change is needed based on a sensor value, whereby the sensor unit (106) is configured to capture pressure, pH value and/or the conductivity value of the expanding agent (114), and has an evaluation unit to determine whether a change is needed.

12. Apparatus according to one of Claims 9 to 11, whereby an insertion aid (116) is mounted to the baseplate (105) for the walls that expand (113) because of the expanding agent (114) during fluid collection.

13. Apparatus according to one of Claims 9 to 12, whereby a valve in the form of a synthetic cusp (118) is mounted to the baseplate (105), which is designed to be opened by the expanding agent (114) behind the walls (113) during fluid collection and to close the stoma after removing the sealing cap with foil and fluid content.

14. Apparatus according to one of the above Claims, whereby the cover is connected to an umbrella-like element (123) through a connector (120), which extends into the intestine and is designed to unfold at the end of the connector opposite the cover through the internal pressure in the intestines or through a support and to inhibit leakage of fluid.

15. Apparatus according to one of the above Claims, whereby the apparatus includes an inflatable balloon introduced into the stoma (122), which is designed to be ventilated and vented in order to optionally seal the stoma (3).

16. Apparatus according to one of the above Claims, whereby a cylindrical receptacle leading into the urostoma is provided on the inside of the cover (101, 102), which has a first chamber leading to the intestinal lumen (125a) that is designed to be inflatable, and a second chamber (125b) adjoining the first chamber (125a) that contains an expanding agent (114) for collecting the content leaving the urostoma (3).

17. Apparatus according to one of the above Claims, whereby the cover and/or the baseplate (102) are at least partially coated with a material that counteracts odors and/or the adhesion of dirt particles.

## Revendications

1. Dispositif pour obturation temporaire iléo- ou urostomacale, comprenant les éléments suivants :
- une plaque de base (105) pour une fixation durable à la périphérie d'un stoma sur la face extérieure de la surface du corps (2),
- au moins un réceptacle disposé temporairement pour récupérer le contenu sortant du stoma (3),
et
- un protecteur qui est lié de façon amovible avec la plaque de base (105), et **caractérisé par**
- **le fait que** le protecteur est formé de façon à constituer un volume récepteur, destiné à recevoir le réceptacle, le protecteur étant constitué d'un bouchon (101) et d'une plaque protectrice (102) qui relie le bouchon (101) dans une ouverture de la plaque protectrice à la plaque de base (105) à travers l'orifice d'un stoma (3).
- le réceptacle est construit sous forme de tube ouvert sur un côté (112a) qui est disposé sur le bouchon (101) et fixé à l'extrémité fermée sur le bouchon (101), et est configuré pour être apte à se dérouler lors du retrait du bouchon (101) de la plaque protectrice (102) et à recevoir le contenu sortant du stoma (3).

2. Dispositif selon la revendication 1 où la plaque de base (105) comprend une encoche (105b) dans laquelle le réceptacle peut être accroché à une extrémité ouverte.

3. Dispositif selon la revendication 1 ou 2 où la plaque protectrice (102) comprend un anneau d'arrêt (103) qui entoure l'ouverture de la plaque protectrice afin de relier une poche de stoma dans la périphérie du stoma (3) de façon soluble et imperméable aux liquides avec la plaque protectrice.

4. Dispositif selon l'une des revendications précédentes où le tube (112a) est formé d'un matériau déformable et se trouve dans le volume récepteur du protecteur après déformation.

5. Dispositif selon l'une des revendications précédentes où le réceptacle du tube (112a) est au moins formé en partie d'une couche de métal (112).

6. Dispositif selon l'une des revendications précédentes où le tube (112a) comporte un boudin annulaire (111) à l'extrémité ouverte, qui est configuré pour être placé dans une encoche périphérique (105b) de la plaque de base (105) et/ou la plaque protectrice (102) et sceller l'ouverture de la plaque de base (105) de l'extérieur.

7. Dispositif selon l'une des revendications précédentes où une section de réception de fluides traversant une paroi perméable aux liquides (113) se trouve dans le tube (112a), qui sert de tube supplémentaire (113a) et est au moins en partie est configuré de façon déformable et/ou pliable.

8. Dispositif selon la revendication 7 où le tube supplémentaire (113a) est plus court que le tube (112a) et dont une extrémité est fixée au bouchon.

9. Dispositif selon l'une des revendications précédentes où un agent gonflant (114) est disposé dans le tube (112a) et/ou dans la zone de réception de fluides.

10. Dispositif selon la revendication 9 où la paroi (113) est configurée pour se dilater lors de l'absorption des fluides par gonflement de l'agent gonflant (114) dans la direction du stoma (3).

11. Dispositif selon l'une des revendications 9 à 10 où le dispositif comprend un capteur (106) qui est configuré pour, en fonction de la valeur affichée, permet d'indiquer si un changement est nécessaire ou non, où le capteur (106) est conçu de façon à détecter le pH et/ou la conductance de l'agent gonflant (114) et affiche une unité d'évaluation pour indiquer si un changement doit être effectué ou non.

12. Dispositif selon l'une des revendications 9 à 11 où un auxiliaire d'insertion (116) pour la paroi (113) qui se dilate lors de l'absorption des fluides par l'agent gonflant (114) est fixé sur la plaque de base (105).

13. Dispositif selon l'une des revendications 9 à 12 où une valve en forme de voile synthétique (118) est fixée sur la plaque de base (105) et est configurée de façon à s'ouvrir derrière la paroi (113) lors de l'absorption des fluides par l'agent gonflant (114) et de fermer le stoma après le retrait du bouchon avec une couche et un contenu fluide.

14. Dispositif selon l'une des revendications précédentes où le protecteur est relié par un joint (120) à un élément en forme de voûte (123), qui pénètre dans l'intestin et est configuré de façon à se déployer à l'extrémité du joint qui se trouve en face du protecteur par pression interne intestinale ou par l'action du support et à empêcher la sortie des fluides.

15. Dispositif selon l'une des revendications précédentes où le dispositif comprend un ballonnet gonflable (122) inséré dans le stoma qui est configuré pour être gonflé et dégonflé de l'extérieur et fermer le stoma (3) le cas échéant.

16. Dispositif selon l'une des revendications précédentes où un réceptacle cylindrique qui s'infiltre dans l'urostomie est prévu à l'intérieur du protecteur (101, 102), ce réceptacle comprenant un premier compartiment (125a) dirigé vers la lumière intestinale (125a) et formé de façon gonflable et un deuxième compartiment (125b) près du premier compartiment (125a), qui contient un agent gonflant source (114) pour la récupération du contenu sortant de l'urostomie (3).

17. Dispositif selon l'une des revendications précédentes où le protecteur et/ou la plaque de base (102) sont au moins partiellement revêtus d'un matériau qui empêche les particules de poussières de s'y coller et/ou la formation d'odeur.
